# EUROPEAN PATENT APPLICATION

(11) **EP 4 397 756 A2**
(43) Date of publication of application: **10.07.2024**
(21) Application number: 21955806.1
(22) Date of filing: 09.12.2021
(51) Int. Cl.: C12N 15/10

(54) **METHOD AND KIT FOR RAPIDLY DETECTING VIRAL GENOME SIZE**

(30) Priority: 30.08.2021 CN 202111005366; 18.10.2021 CN 202111211637
(71) Applicant: Nanjing Bionce Biotechnology Co., Ltd., Nanjing, Jiangsu 210000 (CN)
(72) Inventor: LUO, Guangzuo, Nanjing, Jiangsu 210000 (CN); MENG, Yuan, Nanjing, Jiangsu 210000 (CN)
(74) Representative: karo IP
(86) International application number: PCT/CN2021/136643
(87) International publication number: WO 2023/029278

(57) **Abstract**

The present disclosure discloses a method for rapidly detecting viral genome size based on an agarose gel electrophoresis technology, and belongs to the technical field of virus detection. The kit for detecting the genome size comprises a lysis buffer and a sample loading buffer solution, and comprises the following raw material components: 5% lauryl sodium sulfate, 0.5 M sodium chloride, 50 mM ethylenediamine tetraacetic acid and 6X DNA gel sample loading buffer dye. By adopting the detection method provided by the present disclosure, a plurality of tedious steps of the existing method are omitted, a clearer gel imaging result can be obtained, the detection time is greatly shortened, and the method is particularly suitable for rapidly detecting virus genome size of parvoviridae family. The present disclosure has important application value, and is more in line with the needs of virus detection and quality control research work in the field of gene therapy drug development.

## Description

### TECHNICAL FIELD

The present disclosure belongs to the field of molecular biology, and in particular to a kit for rapidly detecting a viral genome and a detection method thereof.

### BACKGROUND

In recent years, gene therapy has made breakthroughs. In order to ensure the efficacy and stability of gene drugs, quality control of the gene drugs (namely recombinant viruses) is extremely important. A recombination adeno-associated virus (rAAV) has been widely used in the field of gene therapy for decades and has been widely used for treating eye diseases, neuromuscular diseases, central nervous system diseases and the like, which is used as a safe and efficient gene introduction vector for gene therapy. Current production methods for recombination adeno-associated virus gene drugs are greatly improved than that used 20 years ago, but improvement of quality control methods is very limited, which may be related to a slow commercial progress of the gene therapy in the past.

As well known, AAV consists of protein capsid and single-stranded genomic DNA. Previous studies have shown that when the genomic DNA of the adeno-associated virus is extracted by conventional methods, the DNA obtained is positive single-stranded DNA or negative single-stranded DNA. Traditional methods for detecting genome size of the adeno-associated virus are mainly achieved by Southern blot. The methods have tedious steps and high costs, and require use of radioactive isotopes. Thus, the methods are not suitable for being used as quality control methods for gene drugs. In addition, since the DNA is single-stranded, a variety of different secondary structures are easily formed, leading to an unstable migration rate on conventional agarose gel, so that the methods cannot be used as effective quality control means, accordingly, the molecular weight can be determined by using denatured agarose gel electrophoresis after the DNA is changed into a single-stranded form, and an experimental method becomes very tedious. Moreover, since denatured gel needs to be used, requirements for experimental conditions are also increased. At present, commonly used quality control methods include silver staining for detecting protein capsid and real-time quantitative PCR for detecting the genome quantity of viruses. However, there are no simple and rapid methods for visualizing genomic DNA of viruses, and the quality control of the genomic DNA is used to detect whether the gene drugs are approved for clinical application. Therefore, it is urgent to develop a method that is simple and easy to operate.

### SUMMARY

The present disclosure aims to overcome the disadvantages of the prior art and provide a rapid detection kit capable of rapidly, efficiently and simply detecting viral genome size of Parvoviridae family under conventional experimental conditions and a detection method.

In order to achieve the foregoing purposes, the present disclosure provides the following technical schemes.

A method for rapidly detecting viral genome size includes: changing genomic single-stranded DNA of a virus into more stable double-stranded DNA, and then performing detection by agarose gel electrophoresis, where the virus is selected from viruses of Parvoviridae family.

As a preference of the present disclosure, the method includes: adding a lysis buffer or a diluent thereof into a virus sample, subjecting virus capsid in the sample to lysis under suitable conditions, and then performing electrophoresis analysis using agarose gel; the lysis buffer, with water as a solvent, is selected from any one of the following types:
(1) a detergent;
(2) a detergent and NaCl;
(3) a detergent, NaCl and a chelating agent; and
(4) a detergent, NaCl, a chelating agent and other additives; and
after the lysis buffer is added, a final concentration of the detergent in a system is 0.01%-0.1%, a final concentration of the NaCl in the system is 0 M-0.2 M, and a final concentration of the chelating agent in the system is 0 mM-6 mM.

As a further preference of the present disclosure, the detergent is selected from mild detergents, preferably sodium dodecyl sulfate (SDS), polysorbate 20 (Tween-20), TritonX 100, Proteinase K or mixtures thereof, and further preferably the SDS.

As a further preference of the present disclosure, the chelating agent is ethylenediamine tetraacetic acid (EDTA).

As a more further preference of the present disclosure, a final concentration of the SDS in the system is 0.03%-0.1%, most preferably 0.05%.

As a more further preference of the present disclosure, the final concentration of the NaCl in the system is 0.02 M-0.08 M, preferably 0.05 M.

As a more further preference of the present disclosure, a final concentration of the EDTA in the system is 3 mM-6 mM, preferably 5 mM.

In some preferred embodiments of the present disclosure, the lysis buffer includes 0.5% SDS, 0.5 M NaCl and 50 mM EDTA.

As a preference of the present disclosure, the suitable conditions include cooling on ice or at room temperature after heating treatment at 70°C-100°C. A heating temperature of the present disclosure is an appropriate temperature for protein lysis.

As a further preference of the present disclosure, the suitable conditions include cooling at room temperature for 10 min-15 min after heating at 90°C-100°C for 10 min-15 min, preferably, cooling at room temperature for 10 min after heating at 95°C for 10 min.

The virus of the present disclosure is preferably an adeno-associated virus.

A virus lysis buffer is selected from any one of the following types:
(1) a 0.01%-1% detergent;
(2) a 0.01%-1% detergent and 0.01 M-2 M NaCl; and
(3) a 0.01%-1% detergent, 0.01 M-2 M NaCl and a 1.5 mM-100 mM chelating agent.

As a preference of the present disclosure, the detergent is selected from mild detergents, preferably sodium dodecyl sulfate (SDS), polysorbate 20 (Tween-20), TritonX 100, Proteinase K or mixtures thereof, and further preferably the SDS.

As a preference of the present disclosure, the chelating agent is EDTA.

As a further preference of the present disclosure, a concentration of the SDS is 0.3%-1%, most preferably 0.5%.

As a further preference of the present disclosure, a concentration of the NaCl is 0.2 M-0.8 M, furthermore preferably 0.5 M.

As a further preference of the present disclosure, a concentration of the EDTA is 30 mM-60 mM, furthermore preferably 50 mM.

As an optimal preference of the present disclosure, the lysis buffer includes 0.5% SDS, 0.5 M NaCl and 50 mM EDTA.

A kit for detecting viral genome size is provided, where the kit includes the virus lysis buffer of the present disclosure.

As a preference of the present disclosure, the lysis buffer includes 0.5% SDS, 0.5 M NaCl and 50 mM EDTA.

As a preference of the present disclosure, the kit further includes a 6X DNA gel sample loading buffer dye.

The kit of the present disclosure may be supplemented with tubes, microporous plates and test strips for mixing various components as required, instruction information for recording a use method and the like. A detection system may be an instrument with fluorescence reading, chemiluminescence reading, radiation reading and other functions. Optionally, the detection system further includes a computer and detection analysis software.

Traditional methods for detecting the genome size of the adeno-associated virus are mainly achieved by Southern blot. The methods have tedious steps and high costs, and require use of radioactive isotopes. Thus, the methods are not suitable for being used as quality control methods for gene drugs. In addition, since the DNA is single-stranded, a variety of different secondary structures are easily formed, leading to an unstable migration rate on conventional agarose gel, so that the methods cannot be used as effective quality control means, accordingly, the molecular weight can be determined by using denatured agarose gel electrophoresis after the DNA is changed into a single-stranded form, and an experimental method becomes very tedious. Moreover, since denatured gel needs to be used, requirements for experimental conditions are also increased. The kit of the present disclosure mainly has the following advantages. The kit is rapid, efficient and simple, and can maintain the stability of single-stranded DNA. According to the present disclosure, common experimental reagents, materials and method are used, which can detect the genome size of the adeno-associated virus under conventional experimental conditions, and the method is the only effective means for detecting the genome size of adeno-associated virus gene drugs at present.

### Beneficial effects are as follows.

(1) The method and the kit for detecting the genome of the adeno-associated virus provided by the present disclosure can provide clearer electrophoresis imaging results, and have the advantages of brightening strips, reducing a background value, reducing sample residual pore channels and the like.
(2) According to the method and the kit for detecting the genome of the adeno-associated virus provided by the present disclosure, materials used are simple and easy to obtain, requirements for an experimental environment are low, and the method and the kit have the advantages of rapidity, simplicity, effectiveness, practicability and the like.

### BRIEF DESCRIPTION OF THE DRAWINGS

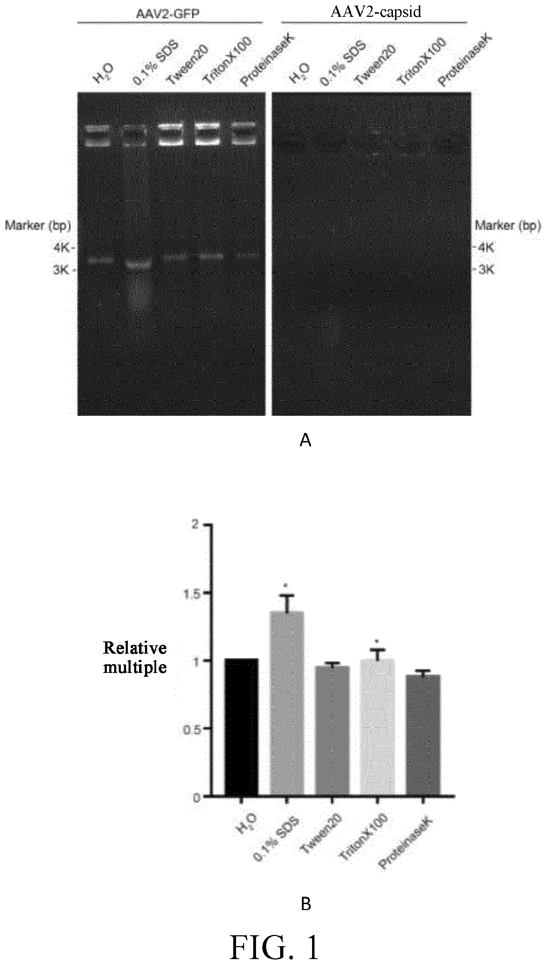

FIG. 1 includes FIG. 1A and FIG. 1B, showing agarose gel electrophoresis results of AAV2-GFP samples treated with lysis buffers 1, 11, 12 and 13, respectively, with water (H₂O) as a blank control and AAV2 capsid as a negative control. FIG. 1A shows electrophoresis results. FIG. 1B shows strip quantification results of an experimental group (AAV2-GFP) in FIG. 1A.

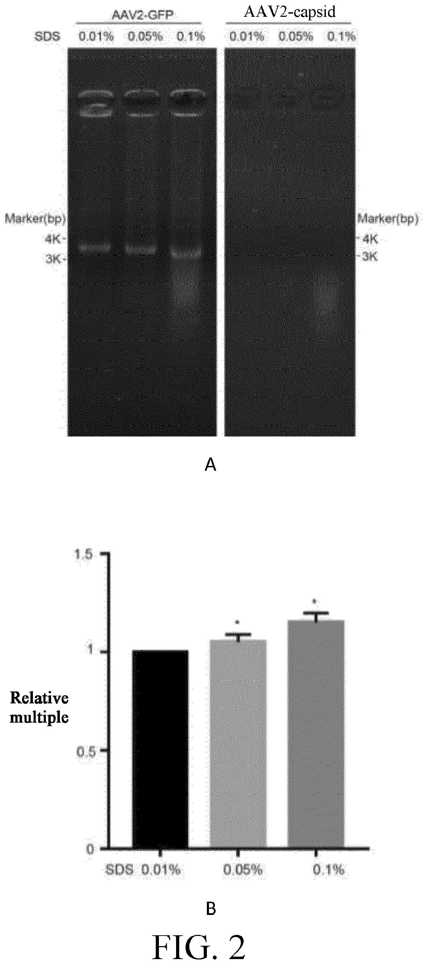

FIG. 2 includes FIG. 2A and FIG. 2B, showing agarose gel electrophoresis results of AAV2-GFP samples treated with lysis buffers 1, 2 and 14, respectively, with AAV2 capsid as a negative control. FIG. 2A shows electrophoresis results. FIG. 2B shows strip quantification results of an experimental group (AAV2-GFP) in FIG. 2A.

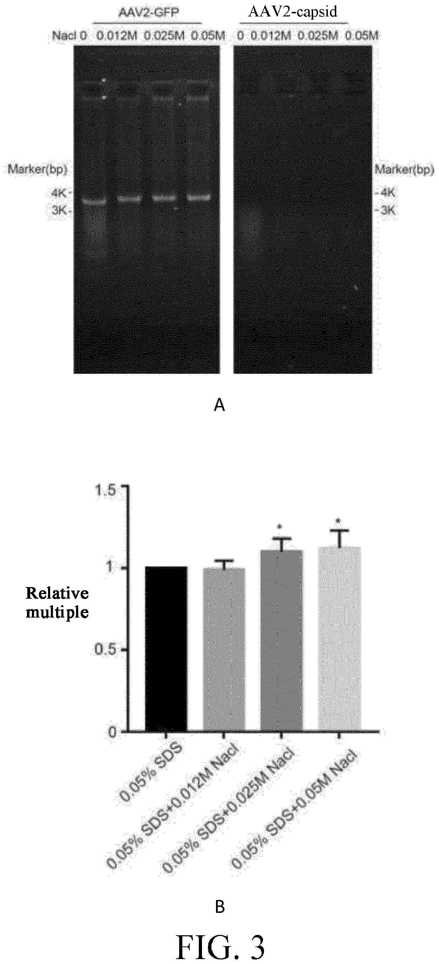

FIG. 3 includes FIG. 3A and FIG. 3B, showing agarose gel electrophoresis results of AAV2-GFP samples treated with lysis buffers 2, 3, 4 and 5, respectively, with AAV2 capsid as a negative control. FIG. 3A shows electrophoresis results. FIG. 3B shows strip quantification results of an experimental group (AAV2-GFP) in FIG. 3A.

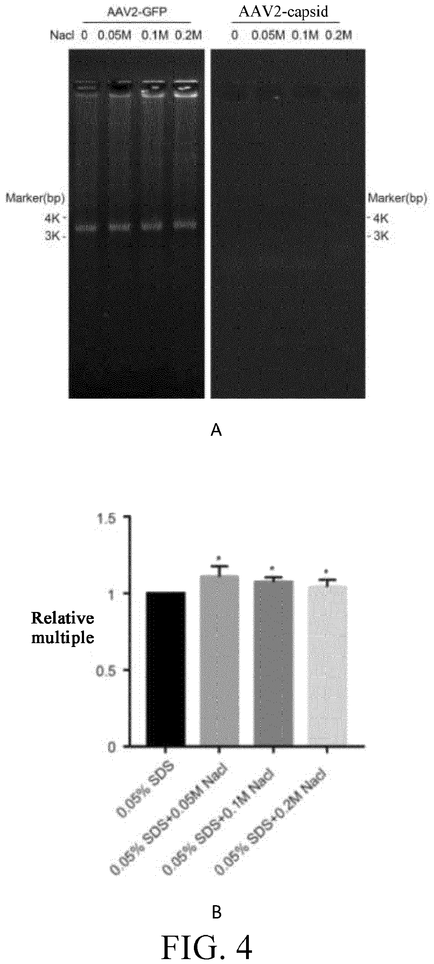

FIG. 4 includes FIG. 4A and FIG. 4B, showing agarose gel electrophoresis results of AAV2-GFP samples treated with lysis buffers 2, 6 and 7, respectively, with AAV2 capsid as a negative control. FIG. 4A shows electrophoresis results. FIG. 4B shows strip quantification results of an experimental group (AAV2-GFP) in FIG. 4A.

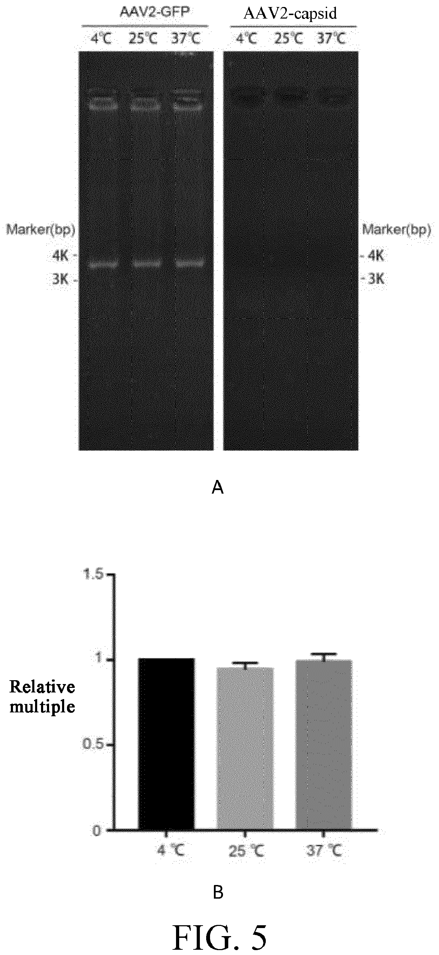

FIG. 5 includes FIG. 5A and FIG. 5B, showing agarose gel electrophoresis results of samples treated with a lysis buffer 5 and rewarmed at 4°C (ice), 25°C (room temperature) or 37°C, respectively, with AAV2 capsid as a negative control. FIG. 5A shows electrophoresis results. FIG. 5B shows strip quantification results of an experimental group (AAV2-GFP) in FIG. 5A.

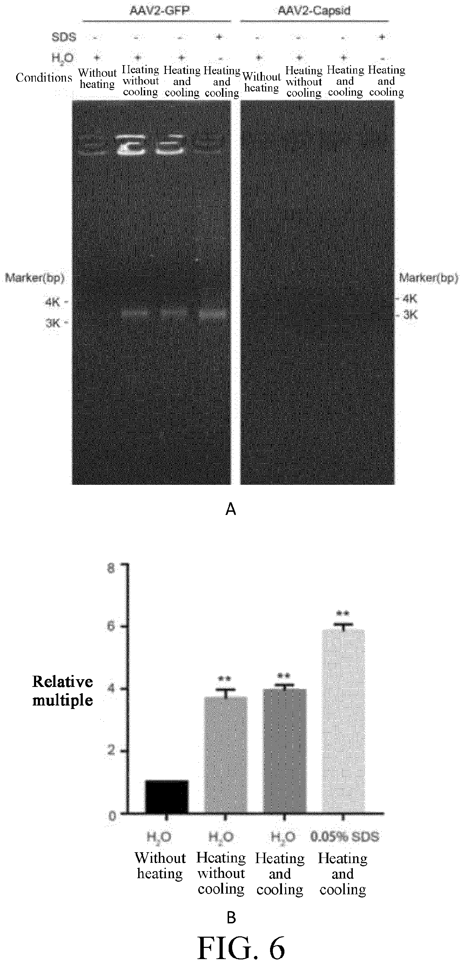

FIG. 6 includes FIG. 6A and FIG. 6B, showing agarose gel electrophoresis results of samples heated at 95°C or not heated, respectively, with AAV2 capsid as a negative control. FIG. 6A shows electrophoresis results, where sample conditions, from left to right, are sequentially as follows: (1) treatment with H₂O without heating, (2) treatment with H₂O and heating at 95°C for 10 min without cooling, (3) treatment with H₂O, heating at 95°C for 10 min and cooling at room temperature for 10 min, and (4) treatment with a lysis buffer 2, heating at 95°C for 10 min and cooling at room temperature for 10 min. FIG. 6B shows strip quantification results of an experimental group (AAV2-GFP) in FIG. 6A.

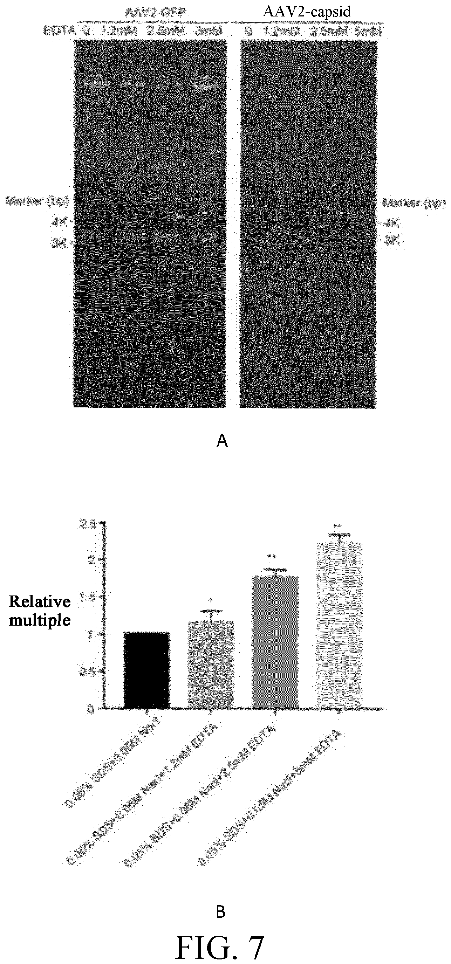

FIG. 7 includes FIG. 7A and FIG. 7B, showing agarose gel electrophoresis results of AAV2-GFP samples treated with lysis buffers 4, 8, 9 and 10, respectively, with AAV2 capsid as a negative control. FIG. 7A shows electrophoresis results. FIG. 7B shows strip quantification results of an experimental group (AAV2-GFP) in FIG. 7A.

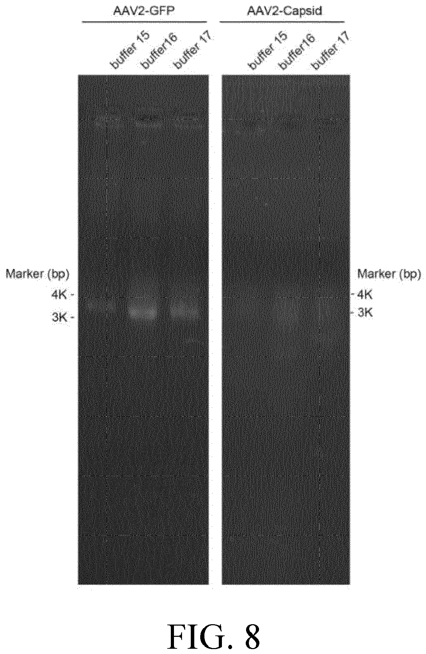

FIG. 8 shows agarose gel electrophoresis results of AAV2-GFP samples treated with lysis buffers 15, 16 and 17, respectively, and results of electrophoresis with AAV2 capsid as a negative control.

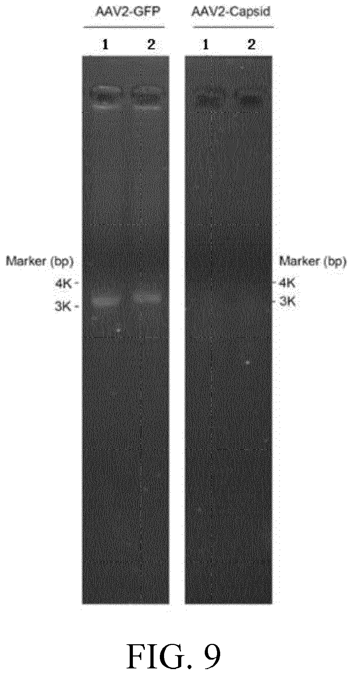

FIG. 9 shows agarose gel electrophoresis results of AAV2-GFP samples treated with a 2X lysis buffer 10 (lane 1) and an undiluted lysis buffer 10 (lane 2), respectively, and results of electrophoresis with AAV2 capsid as a negative control.

### DETAILED DESCRIPTION

The present disclosure is further described below in combination with accompanying drawings and specific examples, but the scope of the protection of the present disclosure is not limited to the following examples. It should also be understood that terms used in the examples of the present disclosure are intended to describe specific embodiments, rather than to limit the scope of the protection of the present disclosure. Without deviating from the spirit and scope of the concept of the present disclosure, all variations and advantages that can be thought of by persons skilled in the art are included in the present disclosure, and the scope of the protection of the present disclosure is defined by the attached claims and any equivalents thereof. In the specification and claims of the present disclosure, the singular forms "a", "one" and "the" include plural forms, unless otherwise explicitly stated herein. Experimental methods without specific conditions specified in the following examples are general knowledge and common knowledge for persons skilled in the art, or are used in accordance with conditions suggested by manufacturers. Unless otherwise specified, all materials and reagents used in the examples are commercially available products.

In the present disclosure, a kit for rapidly detecting a genome of an adeno-associated virus includes:
(1) a 6X DNA gel sample loading buffer dye (6X DNA gel loading purple dye, New England Biolabs, PN B7024); and
(2) a lysis buffer, including components as shown in the following table.

| **Lysis buffer** | | **Component** |
|---|---|---|
| Lysis buffer 1 | 10X buffer 1 | 1% SDS |
| Lysis buffer 2 | 10X buffer 2 | 0.5% SDS |
| Lysis buffer 3 | 10X buffer 3 | 0.5% SDS, 0.1M NaCl |
| Lysis buffer 4 | 10X buffer 4 | 0.5% SDS, 0.5M NaCl |
| Lysis buffer 5 | 10X buffer 5 | 0.5% SDS, 0.25M NaCl |
| Lysis buffer 6 | 10X buffer 6 | 0.5% SDS, 1M NaCl |
| Lysis buffer 7 | 10X buffer 7 | 0.5% SDS, 2M NaCl |
| Lysis buffer 8 | 10X buffer 8 | 0.5% SDS, 0.5M NaCl, 12mM EDTA |
| Lysis buffer 9 | 10X buffer 9 | 0.5% SDS, 0.5M NaCl, 25mM EDTA |
| Lysis buffer 10 | 10X buffer 10 | 0.5% SDS, 0.5M NaCl, 50mM EDTA |
| Lysis buffer 11 | Tween20 | 50% Tween20 |
| Lysis buffer 12 | Triton-X | 50% Triton-X |
| Lysis buffer 13 | Proteinase K | 20 mg/ml Proteinase K |
| Lysis buffer 14 | 10X buffer 3 | 0.1% SDS |
| Lysis buffer 16 | 10X buffer 16 | 0.1% SDS, 0.5M NaCl, 1mM EDTA |
| Lysis buffer 17 | 10X buffer 17 | 1% SDS, 1M NaCl, 3mM EDTA |
| Lysis buffer 18 | 10X buffer 18 | 1% SDS, 2M NaCl, 5mM EDTA |

### Example 1 Test of virus lysis effects of different detergents

Step 1: Lysis buffers 1, 11, 12 and 13 were prepared.

Step 2: Samples were rewarmed at room temperature, and a metal bath was opened.

Step 3: 1% agarose gel was prepared.
(1) 0.25 g of agarose was accurately weighed and poured into a conical flask.
(2) About 25 ml of TAE was measured with a special measuring cylinder (to avoid excessive gel concentration caused by evaporation during heating), poured into the conical flask, and gently shaken for uniform mixing by a small force to prevent drug powder from attaching to a cup wall, which was not conducive to subsequent dissolution.
(3) A mixture obtained after uniform mixing was placed in a microwave oven, and heated for a certain period of time until liquid in the conical flask was boiled and a solution was clear and transparent (the solution was taken out and observed, and heating was continued when particles were observed), where the heating time was not too long so as to prevent the liquid from evaporating completely (a total of about 2 min).
(4) The solution was taken out and slightly cooled, and when the solution was cooled to a temperature acceptable to touch with hands, 1 µl of a dye Gelred was added (when the dye was Goldenview, the addition amount was only 0.1 µl, which might be judged based on experience to avoid a too yellow solution). Since the dye was attached to the head of a gun and was difficult to shoot out at a small addition amount, the dye was shoot out to a cup wall and then dissolved by shaking the conical flask.
(5) Then agarose gel was taken out and usually cooled for 30 min based on experience.
(6) When residual gel was difficult to clean during cleaning of the conical flask, a small amount of water was added, a mixture was heated slightly and directly poured into a waste liquid tank, and finally, the collected gel was wrapped with gloves and discarded or uniformly recycled.

Step 4: An adeno-associated virus to be detected was treated.
(1) The lysis buffers 1, 11, 12 and 13 were diluted to 2X, respectively.
(2) Samples of an experimental group were prepared. 5 µl of AAV2-GFP samples (2×10¹⁰/µl) rewarmed at room temperature were mixed with 5 µl of the 2X lysis buffers 1, 11, 12 and 13 or water, respectively.
(3) Samples of a negative control group were prepared. 5 µl of AAV2 capsid (empty shell) samples rewarmed at room temperature were mixed with 5 µl of the 2X lysis buffers 1, 11, 12 and 13 or water, respectively, and 5 µl of AAV2 capsid samples and 5 µl of the 2X lysis buffers 1, 11, 12 and 13 were used as a blank control group.
(4) The samples in steps (2) and (3) were treated in the metal bath at 95°C for 10 min.
(5) Cooling was performed at room temperature for 10 min.

Step 5: Agarose gel electrophoresis was performed.
(1) 2 µl of a 6X DNA gel loading purple dye was added into the cooled samples.
(2) 3 µl of a 5k Marker was added into a first pore channel.
(3) 8 µl of the samples were sequentially added into pore channels.
(4) Electrophoresis was performed at 100 V and 200 mA for 50 min.
(5) Photographing was performed with a gel imager, where the genome size of AAV2-GFP was about 3.2 kb (FIG. 1A), and the ratio of each lysis buffer of the AAV2-GFP experimental group in FIG. 1A to the blank control group was calculated by ImageJ (FIG. 1B).

Agarose gel electrophoresis results of the samples treated with the lysis buffers 1, 11, 12 and 13 respectively are shown in FIG. 1A. In the AAV2-GFP experimental group, the lysis buffer 1 has more obvious strips than the blank control and the lysis buffers 11, 12 and 13. In addition, except for the lysis buffer 1, a large number of the AAV2-GFP samples are remained in the pore channels under other treatment conditions. FIG. 1B shows electrophoresis strip quantification results of the AAV2-GFP experimental group in FIG. 1A. It can be seen that the lysis buffers 1 and 12 have obvious differences compared with the blank control group, and the lysis buffer 1 obviously has a better effect than the lysis buffer 12. In summary, the experimental results show that the lysis buffer 1 including SDS has an optimal lysis effect on the viral capsid.

### Example 2 Test of the optimal concentration of SDS

Lysis buffers 1, 2 and 14 were prepared, and experimental conditions and methods were used with reference to those in Example 1.

Agarose gel electrophoresis results of samples treated with the lysis buffers 1, 2 and 14 respectively are shown in FIG. 2A. In an AAV2-GFP experimental group, the lysis buffers 2 and 14 have more obvious strips than the lysis buffer 1, but the lysis buffer 14 has a higher strip background value and strip dragging conditions. FIG. 2B shows electrophoresis strip quantification results of the AAV2-GFP experimental group in FIG. 2A. By calculating ratios based on the lysis buffer 1, it can be seen that the lysis buffers 2 and 14 have obvious differences compared with the lysis buffer 1, and the lysis buffer 14 has a slightly better effect than the lysis buffer 2. However, by considering the electrophoresis imaging results comprehensively, the lysis buffer 2 including SDS with a concentration of 0.05% is appropriate.

### Example 3 Test of the optimal concentration of NaCl

Lysis buffers 2-7 were prepared, and experimental conditions and methods were used with reference to those in Example 1.

Agarose gel electrophoresis results of samples treated with the lysis buffers 2-7 respectively are shown in FIG. 3A and FIG. 4A. In AAV2-GFP experimental groups, the samples treated with the lysis buffer 2 have relatively higher strip background values than other experimental groups, and under treatment with the lysis buffers 6 and 7, a large number of the AAV2-GFP samples are remained in pore channels. FIG. 3B and FIG. 4B show electrophoresis strip quantification results of the AAV2-GFP experimental groups in FIG. 3A and FIG. 4A, respectively. By calculating ratios based on the lysis buffer 2, it can be seen that the lysis buffers 4-7 have obvious differences compared with the lysis buffer 2, and the lysis buffer 4 has a relatively better effect. By considering the electrophoresis imaging results comprehensively, the lysis buffer 4 including 0.05% SDS added with 0.05 M NaCl can further improve the strip brightness and reduce strip dragging conditions.

### Example 4 Test of sample cooling conditions

Step 1: A lysis buffer 4 was prepared.

Step 2: Samples were rewarmed at room temperature, and a metal bath was opened.

Step 3: 1% agarose gel was prepared by the same method in Example 1.

Step 4: The samples to be detected were treated.
(1) The lysis buffer 4 was diluted to 2X.
(2) A sample of an experimental group was prepared. 5 µl of an AAV2-GFP sample (2×10¹⁰/µl) rewarmed at room temperature was mixed with 5 µl of the 2X lysis buffer 4.
(3) A sample of a negative control group was prepared. 5 µl of an AAV2 capsid (empty shell) sample rewarmed at room temperature was mixed with 5 µl of the 2X lysis buffer 4.
(4) The samples in steps (2) and (3) were treated in the metal bath at 95°C for 10 min.
(5) The samples in step (4) were cooled on ice for 10 min, cooled at room temperature (25°C) for 10 min, or cooled at 37°C for 10 min, respectively.

Step 5: Agarose gel electrophoresis was performed.
(1) 2 µl of a 6X DNA gel loading purple dye was added into the cooled samples.
(2) 3 µl of a 5k Marker was added into a first pore channel.
(3) 8 µl of the samples were sequentially added into pore channels.
(4) Electrophoresis was performed at a voltage of 100 V and a current of 200 mA for 50 min.
(5) Photographing was performed with a gel imager.

Agarose gel electrophoresis results of the samples treated with the lysis buffer 4 are shown in FIG. 5A, and the AAV2-GFP sample treated with the lysis buffer 4 can have clear strips at 4°C (ice), 25°C (room temperature) or 37°C. FIG. 5B shows electrophoresis strip quantification results of the AAV2-GFP experimental group in FIG. 5A. By calculating ratios at 4°C, it can be seen that the three cooling conditions have no obvious differences, thus proving that the effect of the lysis buffer of the present disclosure is not affected by the temperature of a sample cooling environment.

### Example 5 Test of sample lysis conditions

Step 1: A lysis buffer 2 was prepared.

Step 2: Samples were rewarmed at room temperature, and a metal bath was opened.

Step 3: 1% agarose gel was prepared by the same method in Example 1.

Step 4: The samples to be detected were treated.
(1) The lysis buffer 2 was diluted to 2X.
(2) A sample of an experimental group was prepared. 5 µl of an AAV2-GFP sample (2×10¹⁰/µl) rewarmed at room temperature was mixed with 5 µl of the 2X lysis buffer 2 or water.
(3) A sample of a negative control group was prepared. 5 µl of an AAV2 capsid (empty shell) sample rewarmed at room temperature was mixed with 5 µl of the 2X lysis buffer 2 or water, and 5 µl of an AAV capsid sample and 5 µl of the 2X lysis buffer 2 were used as a blank control group.
(4) The samples in steps (2) and (3) were treated in the metal bath at 95°C for 10 min or were not heated.
(5) Cooling was performed at room temperature (25°C) for 10 min or was not performed.

Step 5: Agarose gel electrophoresis was performed.
(1) 2 µl of a 6X DNA gel loading purple dye was added into the cooled samples.
(2) 3 µl of a 5k Marker was added into a first pore channel.
(3) 8 µl of the samples were sequentially added into pore channels.
(4) Electrophoresis was performed at a voltage of 100 V and a current of 200 mA for 50 min.
(5) Photographing was performed with a gel imager.

Agarose gel electrophoresis results are shown in FIG. 6A, where sample conditions, from left to right, are sequentially as follows: (1) treatment with H₂O without heating; (2) treatment with H₂O and heating at 95°C for 10 min without cooling; (3) treatment with H₂O, heating at 95°C for 10 min and cooling at room temperature for 10 min; and (4) treatment with a lysis buffer 2, heating at 95°C for 10 min and cooling at room temperature for 10 min. In the AAV2-GFP experimental group, the sample treated with the lysis buffer 2 and heated at 95°C for 10 min can have clearest strips without a large number of samples remained in pore channels. FIG. 6B shows electrophoresis strip quantification results of the AAV2-GFP experimental group in FIG. 6A. By calculating ratios based on the conditions of treatment with H₂O without heating, it can be seen that the samples can have obvious differences after being heated, and an optimal effect is achieved when the samples are treated with the lysis buffer 2 and heated at 95°C, proving that the viral protein capsid can be destroyed at a high temperature of 95°C to release single-stranded genomic DNA.

### Example 6 Test of the optimal concentration of EDTA

Lysis buffers 4, 8, 9 and 10 were prepared, and experimental conditions and methods were used with reference to those in Example 1.

Agarose gel electrophoresis results of samples treated with the lysis buffers 4, 8, 9 and 10 respectively are shown in FIG. 7A. In an AAV2-GFP experimental group, the sample treated with the lysis buffer 10 has clearest and brightest strips. FIG. 7B shows electrophoresis strip quantification results of the AAV2-GFP experimental group in FIG. 7A. By calculating ratios based on the lysis buffer 4, it can be seen that the lysis buffers 8-10 have obvious differences compared with the lysis buffer 4, and the lysis buffer 10 has a relatively better effect, thus proving that the addition of EDTA promotes formation of double-stranded DNA and enables strips to be further clear and bright. Therefore, the lysis buffer 10 including 0.05% SDS and 0.05 M NaCl added with 5 mM EDTA is an optimal solution.

### Example 7 Test of lysis buffers in reasonable concentration ranges

Step 1: Lysis buffers 10, 15, 16 and 17 were prepared.

Step 2: Samples were rewarmed at room temperature, and a metal bath was opened.

Step 3: 1% agarose gel was prepared.
(1) 0.25 g of agarose was accurately weighed and poured into a conical flask.
(2) About 25 ml of TAE was measured with a special measuring cylinder (to avoid excessive gel concentration caused by evaporation during heating), poured into the conical flask, and gently shaken for uniform mixing by a small force to prevent drug powder from attaching to a cup wall, which was not conducive to subsequent dissolution.
(3) A mixture obtained after uniform mixing was placed in a microwave oven, and heated for a certain period of time until liquid in the conical flask was boiled and a solution was clear and transparent (the solution was taken out and observed, and heating was continued when particles were observed), where the heating time was not too long so as to prevent the liquid from evaporating completely (a total of about 2 min).
(4) The solution was taken out and slightly cooled, and when the solution was cooled to a temperature acceptable to touch with hands, 1 µl of a dye Gelred was added (when the dye was Goldenview, the addition amount was only 0.1 µl, which might be judged based on experience to avoid a too yellow solution). Since the dye was attached to the head of a gun and was difficult to shoot out at a small addition amount, the dye was shoot out to a cup wall and then dissolved by shaking the conical flask.
(5) Then agarose gel was taken out and usually cooled for 30 min based on experience.
(6) When residual gel was difficult to clean during cleaning of the conical flask, a small amount of water was added, a mixture was heated slightly and directly poured into a waste liquid tank, and finally, the collected gel was wrapped with gloves and discarded or uniformly recycled.

Step 4: An adeno-associated virus to be detected was treated.
(1) The lysis buffers 15, 16 and 17 were diluted to 2X, respectively.
(2) Samples of an experimental group were prepared. 5 µl of AAV2-GFP samples (2×10¹⁰/µl) rewarmed at room temperature were mixed with 5 µl of the 2X lysis buffers 15, 16 and 17, respectively.
(3) Samples of a negative control group were prepared. 5 µl of AAV2 capsid (empty shell) samples rewarmed at room temperature were mixed with 5 µl of the 2X lysis buffers 15, 16 and 17, respectively, and 5 µl of AAV2 capsid samples and 5 µl of the 2X lysis buffers 15, 16 and 17 were used as a blank control group.
(4) The samples in steps (2) and (3) were treated in the metal bath at 95°C for 10 min.
(5) Cooling was performed at room temperature for 10 min.

Step 5: Agarose gel electrophoresis was performed.
(1) 2 µl of a 6X DNA gel loading purple dye was added into the cooled samples.
(2) 3 µl of a 5k Marker was added into a first pore channel.
(3) 8 µl of the samples were sequentially added into pore channels.
(4) Electrophoresis was performed at 100 V and 200 mA for 50 min.
(5) Photographing was performed with a gel imager. Results are shown in FIG. 8. As can be seen from the figure, in the AAV2-GFP experimental group, all the lysis buffers in reasonable ranges can achieve an effect.

### Example 8

Step 1: A lysis buffer 10 was prepared.

Step 2: Samples were rewarmed at room temperature, and a metal bath was opened.

Step 3: Operations were the same as those in Example 7.

Step 4: An adeno-associated virus to be detected was treated.
(1) The lysis buffer 10 was divided into two parts, where one part was not diluted (10X), and the other part was diluted to 2X.
(2) Samples of an experimental group were prepared. Sample in a pore channel 1: 5 µl of an AAV2-GFP sample (1×10¹¹) rewarmed at room temperature was mixed with 5 µl of the 2X lysis buffer 10. Sample in a pore channel 2: 9 µl of an AAV2-GFP sample (1×10¹¹) rewarmed at room temperature was mixed with 1 µl of the undiluted lysis buffer 10.
(3) Samples of a negative control group were prepared. 5 µl of an AAV2 capsid (empty shell) sample rewarmed at room temperature was mixed with 5 µl of the 2X lysis buffer 10 to serve as a negative control of the pore channel 1, and 9 µl of an AAV2 capsid (empty shell) sample rewarmed at room temperature and 1 µl of the undiluted lysis buffer 10 were mixed to serve as a negative control of the pore channel 2.
(4) The samples in steps (2) and (3) were treated in the metal bath at 95°C for 10 min.
(5) Cooling was performed at room temperature for 10 min.

Step 5: Operations were the same as those in Example 7.

Results are shown in FIG. 9. As can be seen from the figure, the molecular weight of viruses with different concentrations in the system can be well determined by the lysis buffer or the diluent thereof.

All documents mentioned in the present disclosure are cited herein for reference in the present application. In addition, it should be understood that after reading the foregoing contents of the present disclosure, persons skilled in the art may make various changes or modifications to the present disclosure, and all the modifications of equivalent forms also fall within the scope defined by the claims of the present application.

## Claims

1. A method for rapidly detecting viral genome size, comprising: changing genomic single-stranded DNA of a virus into more stable double-stranded DNA, and then performing detection by agarose gel electrophoresis, wherein the virus is selected from viruses of Parvoviridae family.

2. The method according to claim 1, wherein the method comprises: adding a lysis buffer or a diluent thereof into a virus sample, subjecting virus capsid in the sample to lysis under suitable conditions, and then performing electrophoresis analysis using agarose gel; the lysis buffer, with water as a solvent, is selected from any one of the following types:
(1) a detergent;
(2) a detergent and NaCl; and
(3) a detergent, NaCl and a chelating agent; and
after the lysis buffer is added, a final concentration of the detergent in a system is 0%-0.1%, a final concentration of the NaCl in the system is 0 M-0.2 M, and a final concentration of the chelating agent in the system is 0 mM-6 mM.

3. The method according to claim 2, wherein the detergent is selected from mild detergents, preferably sodium dodecyl sulfate (SDS), Tween-20, TritonX 100, Proteinase K or mixtures thereof, and further preferably the SDS.

4. The method according to claim 2, wherein the chelating agent is ethylenediamine tetraacetic acid (EDTA).

5. The method according to claim 3, wherein a final concentration of the SDS in the system is 0.03%-0.1%, preferably 0.05%.

6. The method according to claim 2, wherein the final concentration of the NaCl in the system is 0.02 M-0.08 M, preferably 0.05 M.

7. The method according to claim 2, wherein a final concentration of the EDTA in the system is 3 mM-6 mM, preferably 5 mM.

8. The method according to any one of claims 1-7, wherein the lysis buffer comprises 0.5% SDS, 0.5 M NaCl and 50 mM EDTA.

9. The method according to any one of claims 1-7, wherein the suitable conditions comprise cooling on ice or at room temperature after heating treatment at 70°C-100°C.

10. The method according to claim 9, wherein the suitable conditions comprise cooling at room temperature for 10 min-15 min after heating at 90°C-100°C for 10 min-15 min, preferably, cooling at room temperature for 10 min after heating at 95°C for 10 min.

11. A virus lysis buffer, wherein the virus lysis buffer, with water as a solvent, is selected from any one of the following types:
(1) a 0.01%-1% detergent;
(2) a 0.01%-1% detergent and 0.01 M-1 M NaCl; and
(3) a 0.01%-1% detergent, 0.01 M-1 M NaCl and a 1.5 mM-100 mM chelating agent.

12. The virus lysis buffer according to claim 11, wherein the detergent is selected from mild detergents, preferably sodium dodecyl sulfate, polysorbate 20 (Tween-20), TritonX 100, Proteinase K or mixtures thereof, and further preferably the SDS.

13. The virus lysis buffer according to claim 11, wherein the chelating agent is EDTA.

14. The virus lysis buffer according to claim 12, wherein a concentration of the SDS is 0.3%-1%, preferably 0.3%-0.6%, and further preferably 0.5%.

15. The virus lysis buffer according to claim 11, wherein a concentration of the NaCl is 0.2 M-0.8 M, preferably 0.5 M.

16. The virus lysis buffer according to claim 11, wherein a concentration of the EDTA is preferably 30 mM-60 mM, preferably 50 mM.

17. The virus lysis buffer according to any one of claims 11-16, wherein the lysis buffer comprises 0.5% SDS, 0.5 M NaCl and 50 mM EDTA.

18. A kit for detecting viral genome size, comprising the virus lysis buffer according to any one of claims 11-17, wherein a virus is selected from viruses of Parvoviridae family.
